# EUROPÄISCHE PATENTSCHRIFT

(11) **EP 3 285 695 B1**
(45) Veröffentlichungstag und Bekanntmachung des Hinweises auf die Patenterteilung: **20.03.2019**
(21) Anmeldenummer: 16716576.0
(22) Anmeldetag: 15.04.2016
(51) Int. Cl.: A61F 2/64, A61H 3/00, A61F 2/50, A61F 2/68, A61F 2/76

(54) **VERFAHREN ZUR STEUERUNG EINER DÄMPFUNGSVERÄNDERUNG**
METHOD FOR CONTROLLING A DAMPING MODIFICATION
PROCÉDÉ DE CONTRÔLE D'UN CHANGEMENT D'AMORTISSEMENT

(30) Priorität: 24.04.2015 DE 102015106390
(43) Veröffentlichungstag der Anmeldung: 28.02.2018
(73) Patentinhaber: Otto Bock Healthcare Products GmbH, 1070 Wien (AT)
(72) Erfinder: SEIFERT, Dirk, 1070 Wien (AT); PAWLIK, Roland, 1130 Wien (AT); SEYR, Martin, 1040 Wien (AT)
(74) Vertreter: Lins, Martina
(86) Internationale Anmeldenummer: PCT/EP2016/058343
(87) Internationale Veröffentlichungsnummer: WO 2016/169848

(56) Entgegenhaltungen:
- DE-A1-102009 052 887
- DE-A1-102009 052 888
- DE-A1-102009 052 895

## Beschreibung

Die Erfindung betrifft ein Verfahren zur Steuerung einer Dämpfungsveränderung bei einem künstlichen Kniegelenk einer Orthese, eines Exoskelettes oder Prothese, wobei das künstliche Kniegelenk ein Oberteil und ein Unterteil aufweist, die um eine Schwenkachse schwenkbar aneinander befestigt sind, wobei eine Widerstandseinheit zwischen dem Oberteil und dem Unterteil befestigt ist, um einen Widerstand gegen eine Einbeugung oder Streckung des künstlichen Kniegelenks bereitzustellen und der Widerstandseinheit eine Verstelleinrichtung zugeordnet ist, über die der Widerstand verändert wird, wenn ein Sensorsignal einer der Verstelleinrichtung zugeordneten Steuereinheit die Verstelleinrichtung aktiviert, wobei für die Schwungphase der Flexionswiderstand reduziert wird. Kniegelenke für Orthesen, Exoskelette oder Prothesen weisen ein Oberteil mit einem oberen Anschlussteil und einem Unterteil mit einem unteren Anschlussteil auf, die gelenkig miteinander verbunden sind. An dem oberen Anschlussteil sind in der Regel Aufnahmen für einen Oberschenkelstumpf oder eine Oberschenkelschiene angeordnet, während an dem unteren Anschlussteil ein Unterschenkelrohr oder eine Unterschenkelschiene angeordnet sind. Im einfachsten Fall sind das Oberteil und das Unterteil durch ein einachsiges Gelenk verschwenkbar miteinander verbunden.

Um unterschiedliche Anforderungen während der verschiedenen Phasen eines Schrittes oder bei anderen Bewegungen oder Verrichtungen möglichst natürlich nachbilden zu können oder zu unterstützen, ist häufig eine Widerstandseinrichtung vorgesehen, die einen Flexionswiderstand und einen Extensionswiderstand bereitstellt. Über den Flexionswiderstand wird eingestellt, wie leicht sich das Unterteil im Verhältnis zum Oberteil bei einer aufgebrachten Kraft nach hinten schwingen lässt. Der Extensionswiderstand bremst die Vorwärtsbewegung des Unterteils und bildet unter anderem einen Streckanschlag aus.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssenor, ein Neigungssenor und/oder ein Kraft-sensor. In Abhängigkeit von den Sensordaten wird die Position des Extensionsanschlages ermittelt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2009 052 887 A1 beschreibt unter anderem ein Verfahren zur Steuerung eines orthetischen oder prothetischen Gelenkes mit einer Widerstandseinrichtung und Sensoren, wobei über Sensoren während der Benutzung des Gelenkes Zustandsinformationen bereitgestellt werden. Die Sensoren erfassen Momente oder Kräfte, wobei die Sensordaten von zumindest zwei der ermittelten Größen durch eine mathematische Operation miteinander verknüpft werden und dadurch eine Hilfsvariable errechnet wird, die der Steuerung des Beuge- und/oder Streckwiderstandes zugrunde gelegt wird.

Die DE 10 2009 052 895 A1 betrifft ein Verfahren zur Steuerung eines künstlichen Gelenkes einer unteren Extremität mit einer Widerstandseinrichtung, der zumindest ein Aktuator zugeordnet ist. Über den Aktuator kann der Beuge- und/oder Streckwiderstand in Abhängigkeit von Sensordaten verändert werden. Während der Benutzung des Gelenkes werden über Sensoren Zustandsinformationen bereitgestellt. Der Beugewiderstand wird in der Standphase erhöht oder nicht verringert, wenn ein in Richtung auf den vertikal abnehmenden Inertialwinkel eines Unterschenkelteils und ein gleichzeitig belasteter Vorfuß ermittelt wird.

Zur Steuerung des Dämpfungsverhaltens bei der Auslösung der Schwungphase, also während der terminalen Standphase zur Vorbereitung der Einleitung der Schwungphase, werden die Flexionswiderstände noch während der Standphase bei einer Belastung in Flexionsrichtung verringert, was bei einer zu frühen Auslösung zu einem kollabierenden Gelenk führen kann. Der Zeitpunkt der Schwungphasenauslösung, also der Reduzierung des Flexionswiderstandes durch Verringerung der entsprechenden Flexionsdämpfung, wird in der Regel auf der Grundlage einer normalen Schrittdauer errechnet. Ein üblicher Wert der Schrittdauer wird auf der Auswertung einer Vielzahl von Ganganalysen angenommen, auf der Grundlage dieses angenommenen Wertes wird zu einem bestimmten Zeitpunkt nach dem Beginn der Standphase, also dem Heel Strike, standardmäßig eine Schwungphasenauslösung eingeleitet. Problematisch ist eine solche Steuerung, wenn sich die Gehgeschwindigkeiten signifikant verändern oder von dem Standard abweichen. Die Beibehaltung des festgelegten Standardwertes kann bei einem langsamen Gehen dazu führen, dass die Schwungphasenauslösung zu früh beginnt, also der Flexionswiderstand noch während der Standphase zu stark verringert wird.

Aufgabe der vorliegenden Erfindung ist es, ein Verfahren bereitzustellen, mit dem bei einer erhöhten Sicherheit gegen ungewolltes Einbeugen eine flexible Anpassung einer Schwungphasenauslösung auch bei unterschiedlichen Gangsituationen oder Ganggeschwindigkeiten sowie unter Beugelast möglich ist.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Steuerung eines künstlichen Kniegelenkes, insbesondere einer Dämpfungsveränderung eines künstlichen Kniegelenkes einer Prothese, eines Exoskeletts oder Orthese, das ein Oberteil und ein Unterteil aufweist, die um eine Schwenkachse schwenkbar aneinander gelagert sind, wobei eine Widerstandseinheit zwischen dem Oberteil und dem Unterteil angeordnet ist, um einen Widerstand gegen eine Einbeugung oder Streckung des künstlichen Kniegelenkes bereitzustellen und der Widerstandseinheit eine Verstelleinrichtung zugeordnet ist, über die der Widerstand verändert wird, wenn ein Sensorsignal einer der Verstelleinrichtung zugeordneten Steuereinheit die Verstelleinrichtung aktiviert, wobei für die Schwungphase der Flexionswiderstand reduziert wird, sieht vor, dass während des Gehens oder Stehens, insbesondere bei jedem Schritt, der Verlauf zumindest einer Belastungskenngröße, die auf die Orthese oder Prothese einwirkt, erfasst wird, ein Maximum des Belastungskenngrößenverlaufes während der Standphase oder des Stehens ermittelt wird und nach Erreichen des Maximums bei Erreichen eines Schwellwertes der Belastungskenngröße unterhalb des Maximums die Flexionsdämpfung während der Standphase auf ein Schwungphasendämpfungsniveau verringert wird. Durch das Einleiten der Schwungphasenauslösung die damit verbundene Verringerung des Flexionswiderstandes in Abhängigkeit von der Ermittlung eines Maximums einer Belastungskenngröße ist es möglich, den Zeitpunkt der Schwungphasenauslösung und damit den Zeitpunkt der Verringerung der Flexionsdämpfung in Abhängigkeit von dem Verlauf der Belastungskenngröße variabel zu bestimmen. Erst nach Erreichen des Maximums einer Belastungskenngröße und der Feststellung, dass sich die Belastungskenngröße wieder verringert, wird die Flexionsdämpfung verringert. Die Feststellung der Verringerung der Belastungskenngröße kann durch Sensoren und die Auswertung der Sensorsignale über die Belastung erfolgen. Durch die Verringerung der Flexionsdämpfung erst nach den Belastungskenngrößenmaximum wird sichergestellt, dass nicht vor Erreichen eines maximalen Belastungswertes, der zum Einbeugen des künstlichen Kniegelenkes führen kann, eine unerwünschte Verringerung der Flexionsdämpfung stattfindet. Die Schwungphasenauslösung findet somit adaptiv statt, eine Annahme oder Schätzung einer Bewegungsdauer, beispielsweise einer Schrittdauer, ist nicht mehr notwendig. Es ist möglich, auf unterschiedliche Bewegungsabläufe variabel zu reagieren. Eine verkürzte Schrittdauer wird mit dem erfindungsgemäßen Verfahren ebenso ausgeglichen wie eine verlängerte Schrittdauer. Durch die Erfassung der Belastungskenngröße während des Stehens ist es möglich, bereits von dem ersten Schritt an eine Schwungphasenauslösung bereitzustellen, das Belastungsmaximum wird auch während des Stehens ermittelt. Das Verfahren ist zur Steuerung sowohl von Prothesen als auch von Orthesen und Exoskeletten vorgesehen. Wird nachfolgend von Orthesen gesprochen, so gelten die Ausführungen ebenso für die Sonderform der Orthese in Gestalt eines Exoskelettes.

Als Belastungskenngröße sind insbesondere das Knöchelmoment und/oder die Axialkraft auf das Unterteil bevorzugt. Während der Standphase ergibt sich eine glockenförmige Kontur oder ein Doppelhöcker des Knöchelmomentverlaufes sowie des Axialkraftverlaufes über die Zeit. Als Axialkraft wird diejenige Kraft angesehen, die entlang der Längserstreckung des Unterteils wirkt, bei Prothesen entlang der Längserstreckung des Unterschenkelrohrs, bei Orthesen entlang der Längserstreckung der Unterschenkelschiene. Wird das maximale Knöchelmoment oder die maximale Axialkraft erreicht, wird der Verlauf des Knöchelmomentes oder der Axialkraft weiterhin überwacht, die Aufnahme der Sensordaten wird über den gesamten Schritt und für jeden Schritt fortgesetzt. Die Sensoren erfassen oder ermitteln die jeweilige Belastungskenngröße über die gesamte Schrittdauer, also von Heel Strike bis Heel Strike und während des gesamten Gehens. Wird ein festgelegter Grenzwert der abnehmenden Belastungskenngröße erreicht, zum Beispiel ein Wert zwischen 95 % und 50 % der Maximalbelastung, wird die Flexionsdämpfung verringert und eine Schwungphasenauslösung eingeleitet. Die Verringerung des Widerstandes erfolgt dann durch die Aktivierung der Verstelleinrichtung. Durch die Schwungphasenauslösung nach Erreichen der maximalen Belastungskenngröße wird das künstliche Kniegelenk länger gesichert. Ein abnehmender Belastungskenngrößenverlauf wird durch einen Vergleich zweier oder mehrere Sensorwerte, die zeitlich hintereinander erfasst werden, ermittelt.

Der Schwellwert für die Auslösung der Schwungphasendämpfung ist zeitlich variabel und hängt von dem Verlauf der Belastungskenngröße ab. Verringert sich die Belastungskenngröße nach Erreichen des Maximums nur langsam, erfolgt die Freischaltung später, also nach Verstreichen eines längeren Zeitraumes nach dem Heel Strike, fällt die Kurve der Belastungskenngröße steil ab, wird die Schwungphasenauslösung zeitlich früher stattfinden.

Der Schwellwert als solcher kann ebenfalls variabel festgelegt werden. Der Schwellwert hängt beispielsweise von der Gehgeschwindigkeit oder der Gehsituation ab. Bei einem ebenen Gehen kann der Schwellwert beispielsweise bei 90% der Maximallast oder des Maximalwertes der Belastungskenngröße liegen, bei einem Gehen auf der Rampe kann die Schwungphasenauslösung später stattfinden, beispielsweise bei Erreichen von 70% der Maximallast bzw. 70% des Maximalwertes der Belastungskenngröße, bei einem sehr langsamen Gehen kann die Auslöseschwelle bei 50% des Maximalwertes der Belastungskenngröße liegen.

Der Schwellwert kann in Abhängigkeit von der Gehgeschwindigkeit verändert werden. Bei einem schnellen Gehen mit einer Schnellentlastung wird der Schwellwert in die Nähe des Maximalwertes der Belastungskenngröße verlagert, bei einem langsamen Gehen von dem Maximalwert weg. Neben der Axialkraft oder dem Knöchelmoment als Belastungskenngröße kann ebenfalls die Überrollgeschwindigkeit, auch zusätzlich zu dem Knöchelmoment oder der Axialkraft, als Kriterium für die Veränderung des Schwellwertes herangezogen werden. Je schneller das Unterteil, also die Unterschenkelschiene oder das Unterschenkelrohr, von einer entgegen der Gehrichtung in eine in Gehrichtung geneigte Stellung verlagert wird, desto früher erfolgt die Schwungphasenauslösung durch Verringerung des Flexionswiderstandes. Der Schwellwert kann weiterhin in Abhängigkeit von der Entlastungsgeschwindigkeit oder der Geschwindigkeit des Kenngrößenabfalls abhängen. Je größer die Geschwindigkeit des Kraftabfalls oder des Momentenabfalls ist, also je schneller sich die Belastungskenngröße verringert, desto früher wird eine Schwungphasenauslösung bewirkt.

Eine Weiterbildung der Erfindung sieht vor, dass der Schwellwert von einer Winkelstellung einer Prothesenkomponente oder Orthesenkomponente abhängt. Der Schwellwert kann beispielsweise in Abhängigkeit vom Erreichen eines bestimmten Überrollwinkels verändert werden. Wird eine bestimmte Stellung des Unterteils relativ zu der Senkrechten ermittelt, kann auf das Ende eines Bewegungszyklus geschlossen werden. Darüber hinaus kann durch das Erreichen von Winkeln auf die jeweilige Gehsituation geschlossen werden. Wird bei einem Schritt beim Aufsetzen nicht eine volle Kniestreckung erreicht, kann beispielsweise angenommen werden, dass der Patient eine Steigung bergauf geht oder alternierend Treppen steigt, so dass ein bestimmter Schwellwert eingestellt wird, der sich von einem Schwellwert für das Gehen in der Ebene unterscheidet. Gleiches gilt für die Erkennung einer Vorwärtsneigung des Unterteils oder aber auch des Oberteils, als Neigungsrichtung oder Absolutwinkel des Oberteils und/oder des Unterteils zu der Gravitationsrichtung.

Eine Weiterbildung der Erfindung sieht vor, dass die Verringerung der Flexionsdämpfung in Abhängigkeit von dem Verlauf der Belastungskenngröße erfolgt. Verringert sich die Belastungskenngröße sehr schnell, wird auch die Flexionsdämpfung schneller während der Standphase auf ein Schwungphasendämpfungsniveau verringert, erfolgt eher eine langsame Verringerung der Belastungskenngröße, kann auf eine langsame Gehgeschwindigkeit geschlossen werden, was eine langsame Verringerung der Flexionsdämpfung erfordert.

Der Ausgangswert der Flexionsdämpfung vor der Verringerung kann auf einen Wert beziehungsweise Standardwert eingestellt werden, der ein Einbeugen während des Stehens oder in der Standphase sperrt. Der Ausgangspunkt für eine Schwungphasenauslösung mit einer Verringerung der Flexionsdämpfung in der Standphase ist somit ein sperrender Beugewiderstand, so dass bereits von dem ersten Schritt an eine Schwungphaseneinleitung durch Verringerung der Flexionsdämpfung im Bereich der terminalen Standphase erfolgen kann. Der Ausgangswert der Flexionsdämpfung sieht also eine Sperrung in der Standphase oder beim Stehen vor.

Die Verringerung der Dämpfung erfolgt kontinuierlich in Abhängigkeit von der Belastungsgröße, es besteht somit eine direkte Korrelation hinsichtlich der Verringerung der Flexionsdämpfung und dem Verlauf der Belastungskenngröße, insbesondere der Verringerung der Belastungskenngröße.

Das Maximum der Belastungskenngröße liegt grundsätzlich über dem festgelegten Schwellwert, da es anders nicht möglich ist, die Kurve der Belastungskenngröße zu durchlaufen und nach Erreichen des Maximums die Schwungphasenauslösung zu aktivieren. Es wird also ein Schwellwert für den jeweiligen Wert der Belastungskenngröße festgelegt, der überhaupt erreicht werden muss, damit das Verfahren zur Steuerung der Dämpfungsveränderung zur Schwungphasenauslösung aktiviert wird.

Der Schwellwert für die Auslösung der Schwungphasendämpfung kann variabel festgelegt werden und ist abhängig von der Größe der Belastungskenngröße oder des Maximums. Wird nach Erreichen eines Anfangsschwellwertes nur ein vergleichsweise geringes Maximum erreicht, kann sich der Auslöseschwellwert verändern, beispielsweise vergrößern, so dass mit einer schnelleren Verringerung der Flexionsdämpfung eine frühere Schwungphaseneinleitung ausgelöst wird.

Um eine zusätzliche Sicherheit für den Patienten bereit zu stellen, kann die Flexionsdämpfung nach einer Verringerung wieder erhöht werden, wenn der Wert der Belastungskenngröße wieder zunimmt. Durchläuft der Verlauf der Belastungskenngröße nach Erreichen eines Maximums eine Veränderung des Monotonieverhaltens und steigt wieder an, kann die Flexionsdämpfung wieder erhöht werden, da angenommen werden kann, dass der übliche Verlauf der Belastungskenngröße gestört ist, somit ein vom üblichen Gangmuster abweichendes Gangverhalten vorliegt und dadurch eine erhöhte Sicherheit gegen ein ungewolltes Einbeugen des künstlichen Gelenks bereit gestellt werden muss. Eine solche Maßnahme dient dazu, dass das künstliche Gelenk beispielsweise beim Stolpern wieder belastet werden kann, ohne zu kollabieren.

Die Widerstandseinheit kann beispielsweise als Aktuator, beispielsweise als hydraulische, pneumatische, magnetorheologische, magnetische, elektrische, mechanische oder elektromagnetische Widerstandseinheit ausgestaltet sein. Bei hydraulischen oder pneumatischen Widerstandseinheiten werden Überströmkanäle geschlossen, so dass diese Überströmkanäle kein Medium mehr aus einer Extensionskammer in eine Flexionskammer strömen kann. Auf diese Weise kann der Fluss des Mediums zwischen der Extensionskammer und der Flexionskammer ggf. auch vollständig unterbunden werden. Bei mechanischen Widerstandseinrichtungen wird beispielsweise die Reibung soweit erhöht, dass keine weitere Flexion stattfinden kann. Gleiches gilt für elektrisch aktuierte Widerstandseinheiten.

Es können auch Aktuatoren zum Einsatz kommen, die sowohl Energie aktiv in das System einbringen, als auch umgekehrt dem System Energie entziehen und auf diese Weise als Widerstandseinheit wirken. Aktuatoren können beispielsweise als Elektromotoren, hydraulische oder pneumatische Pumpen oder piezoelektrische Elemente ausgebildet sein.

Nachfolgend wird ein Ausführungsbeispiel der Erfindung anhand der Figuren näher erläutert. Es zeigen:
- Figur 1: eine schematische Darstellung einer Prothese; sowie
- Figur 2: einen Belastungskenngrößenverlauf.

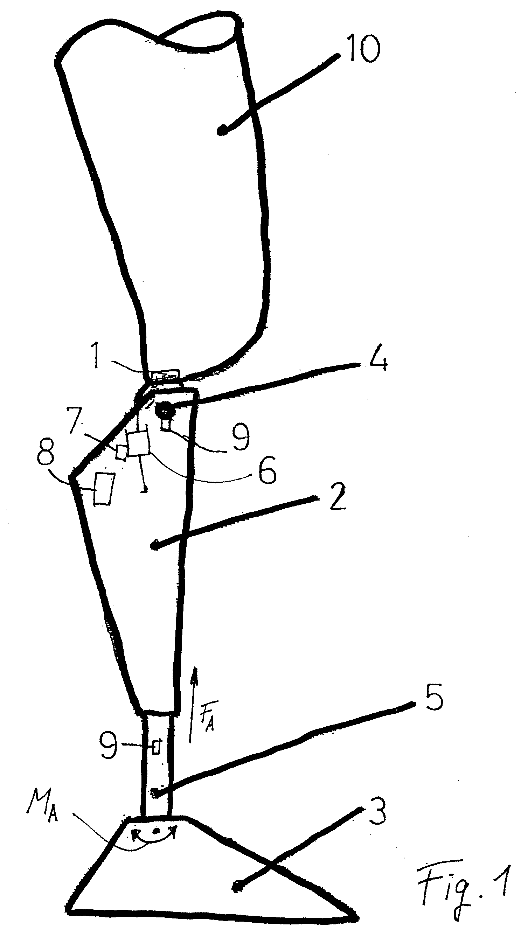

In der Figur 1 ist in einer schematischen Darstellung eine Beinprothese mit einem Oberteil 1 gezeigt, an dem ein Oberschenkelschaft 10 zur Aufnahme eines Oberschenkelstumpfes befestigt ist. An dem Oberteil 1 ist ein Unterteil 2 in Gestalt eines Unterschenkelteils schwenkbar angeordnet. Das Unterteil 2 ist an dem Oberteil 1 um eine Schwenkachse 4 schwenkbar gelagert. Das Unterteil 2 weist ein Unterschenkelrohr 5 auf, an dessen distalen Ende ein Prothesenfuß 3 befestigt ist, in dem eine Einrichtung zur Ermittlung der wirksamen Axialkraft A_{F} auf das Unterschenkelrohr 5 sowie des Knöchelmomentes M_{A}, das um die Befestigungsstelle des Prothesenfußes 3 an dem Unterschenkelrohr 5 wirksam ist, untergebracht sein kann.

In oder an dem Unterteil 2 ist eine Widerstandseinrichtung 6, die beispielsweise als Dämpfer oder Aktuator ausgebildet sein kann, angeordnet, die sich zwischen dem Oberteil 1 und dem Unterteil 2 abstützt, um einen einstellbaren Extensionswiderstand und Flexionswiderstand bereitzustellen. Der Widerstandseinrichtung 6 ist einer Verstelleinrichtung 7 zugeordnet, beispielsweise ein Motor, ein Magnet oder ein anderer Aktuator, über den der jeweilige Widerstand innerhalb der Widersstandseinrichtung 6 verändert werden kann. Ist die Widerstandseinrichtung 6 als Hydraulikdämpfer oder Pneumatikdämpfer ausgebildet, kann über die Verstelleinrichtung 7 der jeweilige Strömungsquerschnitt eines Überströmkanales vergrößert oder verkleinert oder auf andere Weise der Strömungswiderstand verändert werden. Dies kann auch durch Öffnen oder Schließen von Ventilen oder Veränderungen von Viskositäten oder magnetorheologischer Eigenschaften geschehen. Ist die Widerstandseinrichtung als ein Elektromotor im Generatorbetrieb ausgebildet, kann durch Veränderung des elektrischen Widerstandes eine Vergrößerung oder Verringerung der jeweiligen Widerstände gegen eine Flexion oder Extension eingestellt werden.

Um die Verstelleinrichtung 7 aktivieren oder deaktivieren zu können, ist eine Steuerungseinrichtung 8 dem Unterteil 2 zugeordnet, insbesondere in einer Unterschenkelverkleidung angebracht, über die ein entsprechendes Aktivierungs- oder Deaktivierungssignal an die Verstelleinrichtung 7 abgegeben wird. Die Verstelleinrichtung 7 wird auf der Basis von Sensordaten aktiviert oder deaktiviert, die Sensordaten werden von einem oder mehreren Sensoren 9 geliefert, die an dem künstlichen Kniegelenk angeordnet sind. Dies können Winkelsensoren, Beschleunigungssensoren und/oder Kraftsensoren sein. Die Sensoren 9 sind mit der Steuereinrichtung 8 verbunden, beispielsweise per Kabel oder über eine drahtlose Sendeeinrichtung.

Über die Sensoren 9 wird der gesamte Schrittzyklus von dem Fersenstoß (Heel Strike) bis zum erneuten, nachfolgenden Heel Strike HS überwacht, somit auch die gesamte Schwungphase mit der Schwungphasenextension und der Schwungphasenflexion.

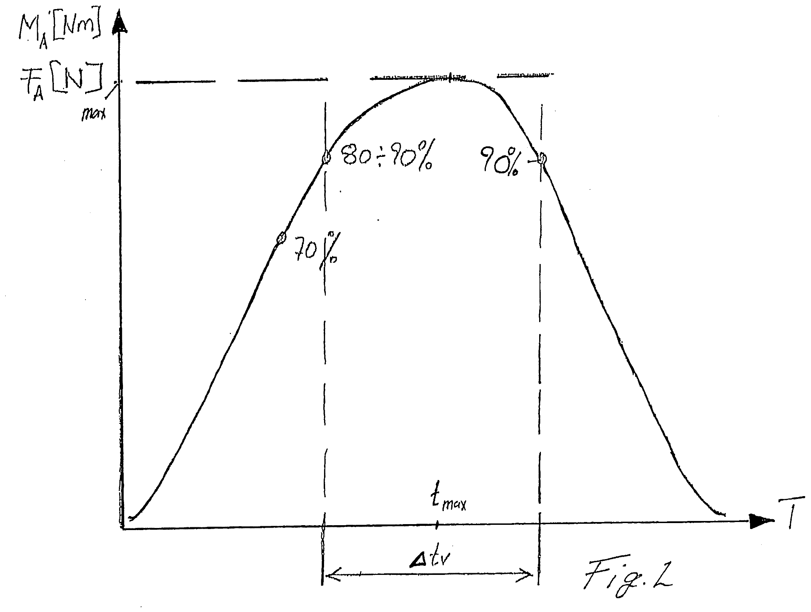

Figur 2 zeigt den Verlauf zweier Belastungskenngrößen, nämlich das Knöchelmoment M_{A} und der Axialkraft F_{A}. Die Axialkraft F_{A} wirkt auf das Unterteil 2 in Richtung der Längserstreckung des Unterteils, das Knöchelmoment M_{A} wirkt im Bereich eines Prothesenfußes 3 oder eines Fußteils einer Orthese. Der Verlauf der Belastungskenngrößen ist über die Zeit T dargestellt. Es ergibt sich ein im Wesentlichen glockenförmiger Verlauf der Belastungskenngrößen M_{A} und F_{A}. Aus dem Stand der Technik ist bekannt, vor Erreichen eines Maximums des Knöchelmoments M_{A} oder der Axialkraft F_{A} die Standphasendämpfung zu reduzieren, um eine Schwungphasenauslösung bewirken zu können. Die Auslösewerte hierfür liegen bei 70% bis 90% vor Erreichen des Maximalwertes M_{Amax} oder F_{Amax} der Belastungskenngröße. Die Auslöseschwellen gemäß dem Stand der Technik sind zeitlich determiniert und liegen vor dem Zeitpunkt tₘₐₓ, zu dem der Maximalwert der Belastungskenngröße anliegt oder angenommen wird.

Erfindungsgemäß ist nunmehr vorgesehen, erst nach Erreichen des Maximums M_{Amax}, F_{Amax} eine Flexionsdämpfungsreduzierung zu veranlassen. Hierzu werden mit einer hohen Abtastrate über den gesamten Schrittzyklus während des Gehens die Belastungskenngrößen über Sensoren 9 erfasst. Ebenso ist es möglich, Winkelgrößen, Winkelgeschwindigkeiten oder Positionsgrößen wie Absolutwinkel zu erfassen und den Verlauf dieser Kenngrößen auszuwerten. Nach Erreichen eines Maximums der Belastungskenngröße oder Belastungskenngrößen wird vor Erreichen der terminalen Standphase und vor der Zehen-ablösung die Flexionsdämpfung reduziert, um ein Einbeugen des künstlichen Kniegelenkes zu ermöglichen und ein an das natürliche Gehen angenähertes Bewegungsmuster bereitstellen zu können. Das Kriterium für eine Freischaltung, also eine Reduzierung der Flexionsdämpfung, ist somit zunächst das Erreichen des Maximalwertes der Belastungskenngröße, beispielsweise des maximalen Knöchelmoments M_{Amax} und/oder der maximalen Axialkraft F_{Amax}. Anschließend wird der weitere Verlauf der Belastungskenngröße überwacht bzw. weiter ermittelt und überprüft, ob ein vorher festgelegter, jedoch variabler Schwellwert, der zeitlich von dem Zeitpunkt tₘₐₓ unabhängig ist, erreicht wird. Der Schwellwert ist nicht zeitlich determiniert, sondern nur von dem Verlauf der Belastungskenngröße abhängig. Eine quantitative Festlegung des Maximalwertes ist ebensowenig notwendig wie die Abschätzung einer Schrittdauer oder Belastungsdauer. Sobald der festgelegte oder auf der Grundlage anderer Sensorwerte bestimmte Schwellwert erreicht wird, kann eine Schwungphasenauslösung durch Verringerung der Flexionsdämpfung erfolgen. Für das Gehen in der Ebene ist ein üblicher Wert 90% der Maximallast bzw. des Maximalmomentes, dieser Wert ist jedoch abhängig von Gehgeschwindigkeit, der Beschaffenheit des Untergrundes sowie der Verwendung von Gehhilfen. Der Schwellwert kann zwischen 95% des Maximalwertes der Belastungskenngröße und 50% des Maximalwertes der Belastungskenngröße variieren. Der Schwellwert muss somit keine fest eingestellte Größe haben, die Stellgröße kann variieren. Durch das Auslösen der Schwungphase nach Erreichen des Maximalwertes der Belastungskenngröße wird für einen längeren Zeitraum, nämlich Δtᵥ das künstliche Kniegelenk länger gesichert. Die Gefahr eines kollabierenden Gelenkes durch vorzeitiges Auslösen der Schwungphase, beispielsweise bei einem sich verzögernden Schritt, wird dadurch zumindest verringert.

Zur Festlegung des Schwellwertes kann die Entlastungsgeschwindigkeit oder die Überrollgeschwindigkeit während des Gehens herangezogen werden. Ebenso ist es möglich, den Überrollwinkel, die Gehrichtung, was durch eine Auswertung der Winkelveränderung des Unterteils zu der Senkrechten ermittelt werden kann, die Qualität des Winkels, also ob eine Vorwärtsneigung oder eine Rückwärtsneigung vorliegt, sowie den Absolutwinkel im Raum zur Veränderung des Schwellwertes einzusetzen und während des Gehens den jeweiligen Schwellwert neu festzulegen. Dadurch kann eine sichere und an das jeweilige Gehverhalten angepasste Schwungphasenauslösung erreicht werden.

Bei einer Schnellentlastung wird die Schwungphase früher ausgelöst als bei einer langsamen Entlastung, was insbesondere bei einem langsamen Gehen eine Erhöhung der Sicherheit gegen unerwünschtes Einbeugen bereitstellt. Alle Ausführungen, die sich auf eine Prothese beziehen gelten entsprechend für eine Orthese. Statt eines Unterschenkelrohres wird dann eine Unterschenkelschiene eingesetzt. Die Festlegung an einem Patienten erfolgt nicht über einen Schaft, sondern über Gurte, Manschette oder dergleichen an dem vorhandenen Bein.

## Patentansprüche

1. Verfahren zur Steuerung einer Dämpfungsveränderung bei einem künstlichen Kniegelenk einer Orthese, eines Exoskelettes oder Prothese, wobei das künstliche Kniegelenk ein Oberteil (1) und ein Unterteil (2) aufweist, die um eine Schwenkachse (4) schwenkbar aneinander befestigt sind, wobei eine Widerstandseinheit (6) zwischen dem Oberteil (1) und dem Unterteil (2) befestigt ist, um einen Widerstand gegen eine Einbeugung oder Streckung des künstlichen Kniegelenks bereitzustellen und der Widerstandseinheit (6) eine Verstelleinrichtung (7) zugeordnet ist, über die der Widerstand verändert wird, wenn ein Sensorsignal einer der Verstelleinrichtung (7) zugeordneten Steuereinheit (8) die Verstelleinrichtung (7) aktiviert, wobei für die Schwungphase der Flexionswiderstand reduziert wird, **dadurch gekennzeichnet, dass** während des Gehens oder Stehens der Verlauf zumindest einer Belastungskenngröße (M_{A}, F_{A}), die auf die Orthese oder Prothese einwirkt, erfasst wird, ein Maximum des Belastungskenngrößenverlaufes während der Standphase oder des Stehens ermittelt wird und nach Erreichen des Maximums bei Erreichen eines Schwellwertes der Belastungskenngröße (M_{A}, F_{A}) unterhalb des Maximums die Flexionsdämpfung während der Standphase auf ein Schwungphasendämpfungsniveau verringert wird.

2. Verfahren nach Anspruch 1, **dadurch gekennzeichnet, dass** Belastungskenngröße das Knöchelmoment (MA) und/oder die Axialkraft (FA) auf das Unterteil (2) verwendet werden.

3. Verfahren nach Anspruch 1 oder 2, **dadurch gekennzeichnet, dass** Schwellwert für die Auslösung der Schwungphasendämpfung zeitlich variabel ist.

4. Verfahren nach einem der voranstehenden Ansprüche, **dadurch gekennzeichnet, dass** der Schwellwert für die Auslösung der Schwungphasendämpfung in Abhängigkeit von der Gehgeschwindigkeit, der Überrollgeschwindigkeit und/oder der Gehsituation festgelegt wird.

5. Verfahren nach einem der voranstehenden Ansprüche, **dadurch gekennzeichnet, dass** der Schwellwert für die Auslösung der Schwungphasendämpfung in Abhängigkeit von der Entlastungsgeschwindigkeit festgelegt wird.

6. Verfahren nach einem der voranstehenden Ansprüche, **dadurch gekennzeichnet, dass** der Schwellwert für die Auslösung der Schwungphasendämpfung in Abhängigkeit von einer Winkelstellung einer Prothesenkomponente oder Orthesenkomponente festgelegt wird.

7. Verfahren nach einem der voranstehenden Ansprüche, **dadurch gekennzeichnet, dass** das Schwellwertniveau für die Auslösung der Schwungphasendämpfung aus einem Bereich zwischen 95% und 50% des Maximalwertes der Belastungskenngröße (M_{A}, F_{A}) ausgewählt wird.

8. Verfahren nach einem der voranstehenden Ansprüche, **dadurch gekennzeichnet, dass** die Verringerung der Flexionsdämpfung in Abhängigkeit von dem Verlauf der Belastungskenngröße erfolgt.

9. Verfahren nach einem der voranstehenden Ansprüche, **dadurch gekennzeichnet, dass** der Ausgangswert der Flexionsdämpfung vor der Verringerung auf einen Wert eingestellt wird, der ein Einbeugen während des Stehens oder in der Standphase sperrt.

10. Verfahren nach einem der voranstehenden Ansprüche, **dadurch gekennzeichnet, dass** die Flexionsdämpfung nach einer Verringerung wieder erhöht wird, wenn der Wert der Belastungskenngröße (M_{A}, F_{A}) wieder zunimmt.

## Claims

1. A method for controlling a damping variation in an artificial knee joint of an orthosis, an exoskeleton or prosthesis, wherein the artificial knee joint has an upper part (1) and a lower part (2), which are fastened to one another in a manner pivotable about a pivot axis (4), wherein a resistance unit (6) is fastened between the upper part (1) and the lower part (2) in order to provide a resistance to flexion or extension of the artificial knee joint and the resistance unit (6) is assigned an adjustment device (7) by means of which the resistance is varied when a sensor signal of a control unit (8) assigned to the adjustment device (7) activates the adjustment device (7), wherein the flexion resistance is reduced for the swing phase, **characterized in that**, when walking or standing, the profile of at least one load characteristic (M_{A}, F_{A}), which acts on the orthosis or prosthesis, is captured, a maximum of the load characteristic profile is ascertained during the stance phase or when standing and, after reaching the maximum, the flexion damping is reduced during the stance phase to a swing phase damping level if a threshold of the load characteristic (M_{A}, F_{A}) below the maximum is reached.

2. The method as claimed in claim 1, **characterized in that** the ankle moment (MA) and/or the axial force (F_{A}) on the lower part (2) are used load characteristic.

3. The method as claimed in claim 1 or 2, **characterized in that** threshold for triggering the swing phase damping is time variable.

4. The method as claimed in any one of the preceding claims, **characterized in that** the threshold for triggering the swing phase damping is set depending on the walking speed, the roll-over speed and/or the walking situation.

5. The method as claimed in any one of the preceding claims, **characterized in that** the threshold for triggering the swing phase damping is set depending on the unloading speed.

6. The method as claimed in any one of the preceding claims, **characterized in that** the threshold for triggering the swing phase damping is set depending on an angle position of a prosthesis component or orthosis component.

7. The method as claimed in any one of the preceding claims, **characterized in that** the threshold level for triggering the swing phase damping is selected from a range between 95% and 50% of the maximum value of the load characteristic (M_{A}, F_{A}).

8. The method as claimed in any one of the preceding claims, **characterized in that** the flexion damping is reduced depending on the profile of the load characteristic.

9. The method as claimed in any one of the preceding claims, **characterized in that** the initial value of the flexion damping prior to the reduction is set to a value which blocks flexion when standing or in the stance phase.

10. The method as claimed in any one of the preceding claims, **characterized in that** the flexion damping after a reduction is increased again when the value of the load characteristic (M_{A}, F_{A}) increases again

## Revendications

1. Procédé pour commander une modification d'amortissement dans une articulation de genou artificielle d'une orthèse, d'un exosquelette ou d'une prothèse,
dans lequel
l'articulation de genou artificielle comprend une partie supérieure (1) et une partie inférieure (2) qui sont fixées l'une à l'autre avec faculté de basculement autour d'un axe de basculement (4),
une unité de résistance (6) est fixée entre la partie supérieure (1) et la partie inférieure (2) pour assurer une résistance à l'encontre de la flexion ou de l'extension de l'articulation de genou artificielle, et
un dispositif de réglage (7) est associé à l'unité de résistance (6), par lequel la résistance est modifiée lorsqu'un signal de capteur d'une unité de commande (8) associée au dispositif de réglage (7) active le dispositif de réglage (7),
la résistance à la flexion est réduite pour la phase pendulaire,
**caractérisé en ce que**
pendant la marche ou la station debout, on détecte l'évolution d'au moins une grandeur caractéristique de charge (M_{A}, F_{A}) qui agit sur l'orthèse ou sur la prothèse, on détermine une valeur maximale de l'évolution de la grandeur caractéristique de charge pendant la phase de station debout, et une fois que la valeur maximale est atteinte, lorsqu'une valeur seuil de la grandeur caractéristique de charge (M_{A}, F_{A}) au-dessous de la valeur maximale est atteinte, l'amortissement de flexion pendant la phase de station debout est réduite à un niveau d'amortissement de phase pendulaire.

2. Procédé selon la revendication 1,
**caractérisé en ce que**
à titre de grandeur caractéristique de charge, on utilise le couple de cheville (MA) et/ou la force axiale (FA) exercée sur la partie inférieure (2).

3. Procédé selon la revendication 1 ou 2,
**caractérisé en ce que**
la valeur seuil pour le déclenchement de l'amortissement en phase pendulaire est variable dans le temps.

4. Procédé selon l'une des revendications précédentes,
**caractérisé en ce que**
la valeur seuil pour le déclenchement de l'amortissement en phase pendulaire est fixée en fonction de la vitesse de marche, de la vitesse de déroulement et/ou de la situation de marche.

5. Procédé selon l'une des revendications précédentes,
**caractérisé en ce que**
la valeur seuil pour le déclenchement de l'amortissement en phase pendulaire est fixée en fonction de la vitesse de décharge.

6. Procédé selon l'une des revendications précédentes,
**caractérisé en ce que**
la valeur seuil pour le déclenchement de l'amortissement en phase pendulaire est fixée en fonction d'une position angulaire d'un composant de la prothèse ou d'un composant de l'orthèse.

7. Procédé selon l'une des revendications précédentes,
**caractérisé en ce que**
le niveau de la valeur seuil pour le déclenchement de l'amortissement en phase pendulaire est choisi dans une plage entre 95 % et 50 % de la valeur maximale de la grandeur caractéristique de charge (M_{A}, F_{A}).

8. Procédé selon l'une des revendications précédentes,
**caractérisé en ce que**
la réduction de l'amortissement de flexion s'effectue en fonction de l'évolution de la grandeur caractéristique de charge.

9. Procédé selon l'une des revendications précédentes,
**caractérisé en ce que**
avant la réduction, la valeur de départ de l'amortissement de flexion est réglée à une valeur qui bloque une flexion pendant la phase de station debout.

10. Procédé selon l'une des revendications précédentes,
**caractérisé en ce que**
après une réduction, l'amortissement de flexion est de nouveau augmentée lorsque la valeur de la grandeur caractéristique de charge (M_{A}, F_{A}) augmente de nouveau.
